(19) Europäisches Patentamt
European Patent Office
Office européen des brevets

(11) Veröffentlichungsnummer: **0 007 440**
**B1**

# (12) EUROPÄISCHE PATENTSCHRIFT

(45) Veröffentlichungstag der Patentschrift: **08.04.81**

(51) Int. Cl.³: **C 07 D 251/04**, C 08 G 18/20

(21) Anmeldenummer: **79102081.1**

(22) Anmeldetag: **25.06.79**

## (54) Hexahydrotriazincarboxylate, ihre Herstellung und Verwendung als Katalysatoren zur Herstellung von Polyisocyanuratkunststoffen.

(30) Priorität: **06.07.78 DE 2829670**

(43) Veröffentlichungstag der Anmeldung: **06.02.80 Patentblatt 80/3**

(45) Bekanntmachung des Hinweises auf die Patenterteilung: **08.04.81 Patentblatt 81/14**

(84) Benannte Vertragsstaaten: **BE DE FR GB IT**

(56) Entgegenhaltungen:
**DE-A-1 769 043**
**DE-A-2 422 335**
**DE-A1-2 545 118**
**DE-A1-2 616 416**
**DE-A1-2 736 799**

(73) Patentinhaber: **BAYER AG, Zentralbereich Patente, Marken und Lizenzen, D-5090 Leverkusen 1, Bayerwerk (DE)**

(72) Erfinder: **Haas, Peter, Dr., Zwengenberger Strasse 43, D-5657 Haan 1 (DE)**
Erfinder: **Blahak, Johannes, Dr., Andreas-Gryphius-Strasse 7, D-5000 Köln 80 (DE)**
Erfinder: **Wiedermann, Rolf, Dr., Schmillenburg 9, D-5060 Bergisch-Gladbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 007 440 B1

# Hexahydrotriazincarboxylate, ihre Herstellung und Verwendung als Katalysatoren zur Herstellung von Polyisocyanuratkunststoffen

Es sind bereits zahlreiche Katalysatoren für die Herstellung von Isocyanuratkunststofffen bekannt geworden. Mit den bekannten Katalysatoren gelang es jedoch bisher nicht befriedigend, das Problem der Schrumpfstabilität, das vorwiegend bei Polyisocyanurat-Schaumstoffen mit längeren Abbindezeiten auftritt, zu lösen.

Das trifft auch zu für die Verwendung von Gemischen bzw. Additionsverbindungen aus Hexahydrotriazinen mit Carbonsäuren und deren Salzen als Katalysatoren für die Herstellung von Polyisocyanuratkunststoffen, wie sie aus der DE-A 2 545 118 bzw. DE-A 2 616 416 bekannt sind.

Überraschenderweise wurde gefunden, daß mit den erfindungsgemäßen Hexahydrotriazincarboxylaten auch bei langen Abbindezeiten schrumpfstabile Polyisocyanurat-Schaumstoffe erhalten werden.

Das Erzielen einer guten Schrumpfstabilität ermöglicht nun auch die oft erwünschte Kombination der erfindungsgemäßen Katalysatoren mit anderen, an sich bekannten Polyurethan-Katalysatoren. Bisher konnten bei der Polyisocyanurat-Schaumstoffherstellung den Isocyanurat-Katalysatoren nur geringe Mengen an Polyurethan-Katalysatoren zugesetzt werden, da sonst Schrumpfprobleme auftreten. Die Kombination von Polyurethankatalysatoren mit den erfindungsgemäßen Katalysatoren ermöglicht eine gezielte Reaktionssteuerung und erlaubt die Einstellung jedes gewünschten Verhältnisses von Start- und Abbindezeit, ohne daß Schrumpfeffekte auftreten.

Gegenstand der vorliegenden Erfindung sind Hexahydrotriazincarboxylate der allgemeinen Formel

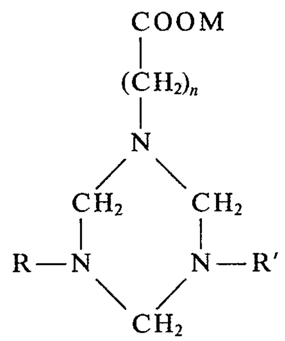

(I)

in der

R und R′ gleich oder verschieden sein können und einen Rest

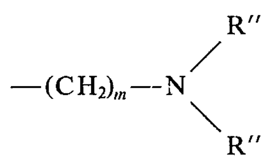

oder einen Rest

$$—(CH_2)_n—COOM$$

bedeuten,

R″ einen $C_1—C_3$-Alkylrest, vorzugsweise einen Methylrest,
m eine ganze Zahl von 2 bis 6, vorzugsweise 2—4,
n eine ganze Zahl von 1 bis 10, vorzugsweise 1—4, und
M ein Alkalimetall, vorzugsweise Na oder K, oder $NR_4'''$, (R‴ H oder $C_1—C_4$-Alkyl) darstellt.

Gegenstand der vorliegenden Erfindung ist ferner ein Verfahren zur Herstellung der obengenannten Hexahydrotriazincarboxylate, dadurch gekennzeichnet, daß man äquimolare Mengen an Formaldehyd und den Verbindungen

$$H_2N—(CH_2)_n—COOM \qquad H_2N—R$$

und

$$H_2N—R'$$

in denen R, R′ und M die obigen Bedeutungen aufweisen, miteinander umsetzt.

Die Erfindung betrifft auch die Verwendung der obengenannten Hexahydrotriazine, gegebenenfalls in Kombination mit anderen bekannten Polyisocyanurat- oder Polyurethan-Katalysatoren, als Katalysatoren zur Herstellung von Polyisocyanurat-Kunststoffen.

Erfindungsgemäß kommen beliebige Verbindungen der allgemeinen Formel I in Frage, z. B. die entsprechenden Alkalimetallsalze wie Li-, Na-, K-, Rb-, Cs-Salze oder Ammoniumsalze, wobei jedoch die Na- und/oder K-Salze bevorzugt sind.

Erfindungsgemäße Hexahydrotriazincarboxylate sind z. B.:

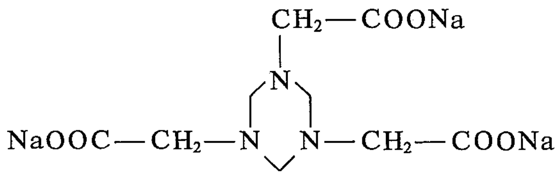

bevorzugt

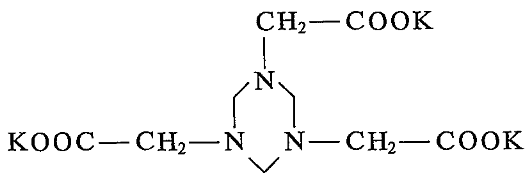

bevorzugt

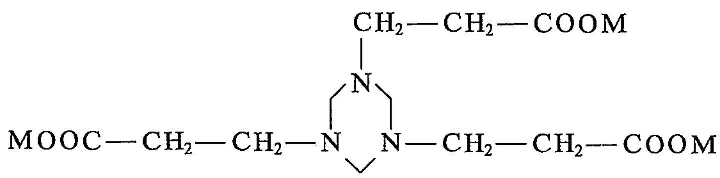

M = Na, K

bevorzugt

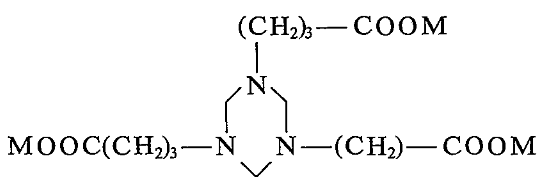

M = Na, K

bevorzugt

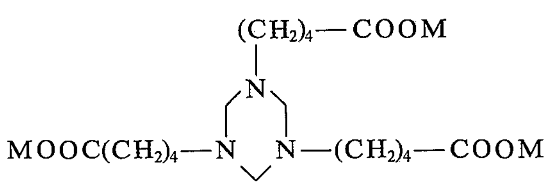

M = Na, K

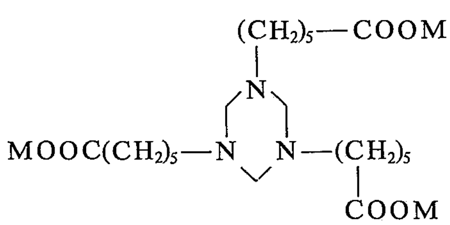

M = Na, K

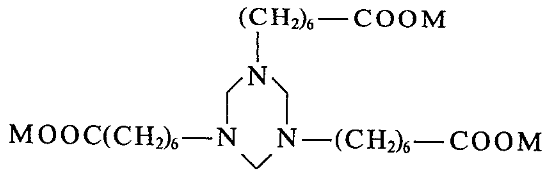

M = Na, K

bevorzugt

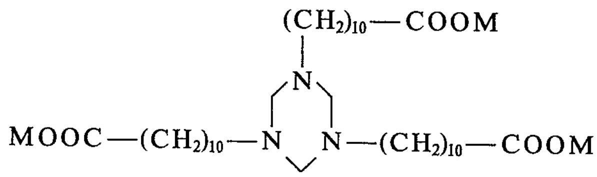

M = Na, K

bevorzugt

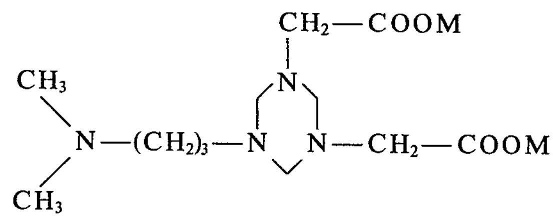

M = Na, K

bevorzugt

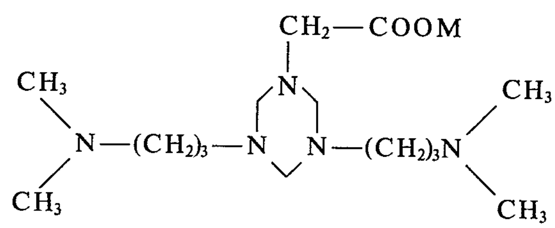

M = Na, K

bevorzugt

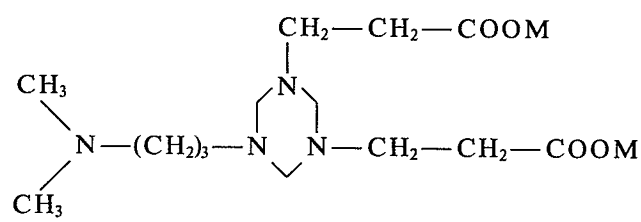

M = Na, K

bevorzugt

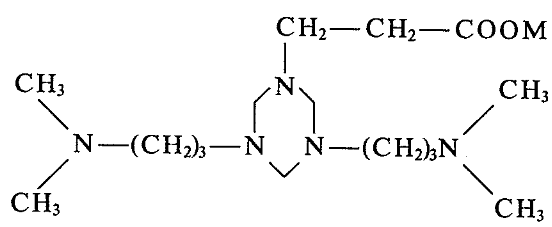

M = Na, K

bevorzugt

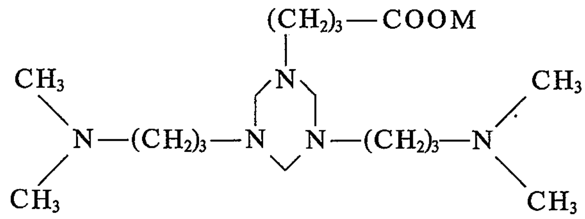

M = Na, K

bevorzugt

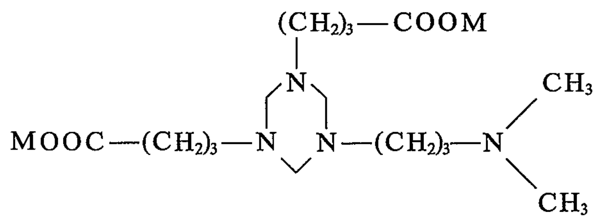

M = Na, K

bevorzugt

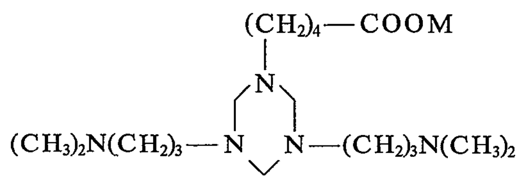

M = Na, K

bevorzugt

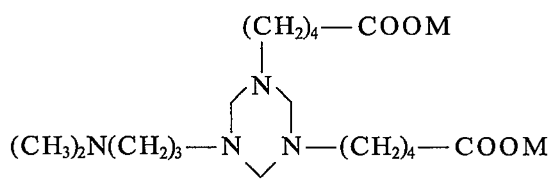

M = Na, K

bevorzugt

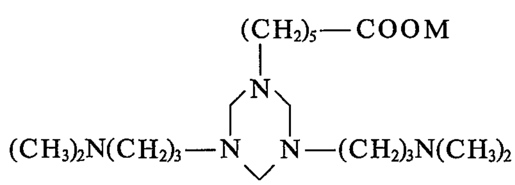

M = Na, K

bevorzugt

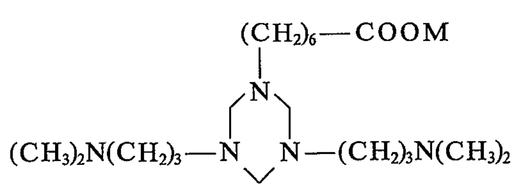

M = Na, K

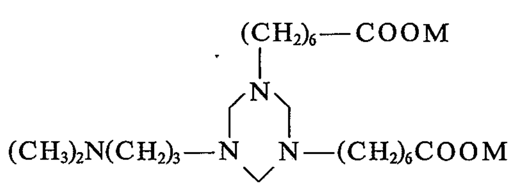

M = Na, K

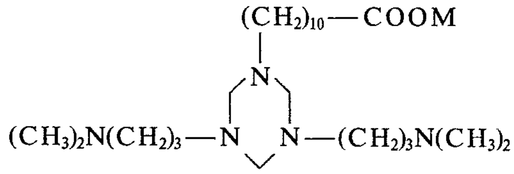

M = Na, K

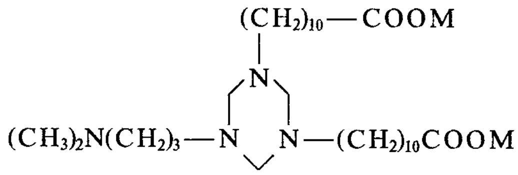

M = Na, K

Die Herstellung der Verbindungen der allgemeinen Formel I erfolgt nach Analogieverfahren durch Kondensation von Formaldehyd mit den bereits genannten Verbindungen

$$H_2N-(CH_2)_n-COOM \qquad H_2N-R'$$

und

$$H_2N-R'$$

in denen M, R, R' und n die schon genannte Bedeutung besitzen. Die Reaktionspartner werden dabei in der Regel in äquimolaren Mengen eingesetzt. Es kann dabei bei Raumtemperatur oder erhöhter Temperatur gearbeitet werden, z. B. bei 50°C. Es ist auch möglich, bei der Kondensation inerte Lösungsmittel wie Wasser, Äthanol, oder Isopropanol mitzuverwenden. Formaldehyd wird im allgemeinen in Form seiner wäßrigen Lösung oder als Paraformaldehyd oder Trioxan eingesetzt. Als Verbindungen

$$H_2N-(CH_2)_n-COOM$$

kommen grundsätzlich alle unter diese Definition fallenden Verbindungen in Frage, bevorzugt sind Glycin, β-Aminopropionsäure, γ-Aminobuttersäure, ε-Aminocapronsäure und 11-Aminoundecansäure. Auch als Verbindungen $H_2N-R$ und $H_2N-R'$ kommen grundsätzlich alle unter diese Definition fallenden Verbindungen in Frage, insbesondere z. B. γ-Dimethylamino-propylamin, β-Dimethylamino-äthylamin sowie δ-Dimethylamino-butylamin.

Die Kondensation zum Hexahydrotriazin erfolgt durch z. B. aceotropes Auskreisen von Wasser aus der Reaktionslösung, bevorzugt durch Toluol.

Die erfindungsgemäßen Verbindungen der allgemeinen Formel I werden in der Regel in einer Menge von 0,01—20 Gew.-%, vorzugsweise 0,1—10 Gew.-%, bezogen auf die nachstehend genannten Polyisocyanate, als Katalysator bei der Herstellung von Polyisocyanurat-Kunststoffen eingesetzt, bevorzugt als Lösungen in höherfunktionellen Alkoholen wie Äthylenglykol, Diäthylenglykol, Dipropylenglykol und Tripropylenglykol.

Die Herstellung von Polyisocyanurat-Kunststoffen ist an sich bekannt. Als Ausgangskomponenten hierfür werden verwendet:
aliphatische, cycloaliphatische, araliphatische aromatische und heterocyclische Polyisocyanate, gegebenenfalls (in solchen Mengen, daß mindestens 25 Gew.-% der ursprünglich vorhandenen Isocyanatgruppen in den Polyisocyanaten für eine Trimerisierung frei bleiben) als Ausgangskomponenten Verbindung mit mindestens zwei gegenüber Isocyanaten reaktionsfähigen Wasserstoffatomen von einem Molekulargewicht in der Regel von 400—10 000. Hierunter versteht man neben Aminogruppen, Thiolgruppen oder Carboxylgruppen aufweisenden Verbindungen, vorzugsweise Hydroxylgruppen aufweisende Verbindungen, insbesondere zwei bis acht Hydroxylgruppen aufweisende Verbindungen, speziell solche vom Molekulargewicht 1000 bis 5000, vorzugsweise 800 bis 3000, z. B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind, gegebenenfalls als Ausgangskomponenten Verbindungen mit mindestens zwei gegenüber Isocyanaten reaktionsfähigen Wasserstoffatomen und einem Molekulargewicht von 32 bis 400 und gegebenenfalls Hilfs- und Zusatzmittel. Alle diese Komponenten werden ausführlich in der veröffentlichten Europäischen Patentanmeldung 0 007 440 auf den Seiten 10—31 beschrieben.

## Durchführung des erfindungsgemäßen Verfahrens

Die Reaktionskomponenten werden erfindungsgemäß nach dem an sich bekannten Einstufenverfahren, dem Prepolymerverfahren oder dem Semiprepolymerfahren zur Umsetzung gebracht, wobei man sich oft maschineller Einrichtungen bedient, z. B. solcher, die in der US-Patentschrift 2 764 565 beschrieben werden. Einzelheiten über Verarbeitungseinrichtungen, die auch erfindungsgemäß in Frage kommen, werden im Kunststoff-Handbuch, Band VII, herausgegeben von Vieweg und Höchtlen, Carl-Hanser-Verlag, München 1966, z. B. auf den Seiten 121 bis 205 beschrieben.

Bei der Schaumstoffherstellung kann erfindungsgemäß die Verschäumung auch in geschlossenen Formen durchgeführt werden. Dabei wird das Reaktionsgemisch in eine Form eingetragen. Als Formmaterial kommt Metall, z. B. Aluminium, oder Kunststoff, z. B. Epoxidharz, in Frage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Erfindungsgemäß kann man in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter »overcharging« gearbeitet; eine derartige Verfahrensweise ist z. B. aus den US-Patentschriften 3 178 490 und 3 182 104 bekannt.

Bei der Formverschäumung werden vielfach an sich bekannte »äußere Trennmittel«, wie Siliconöle, mitverwendet. Man kann aber auch sogenannte »innere Trennmittel«, gegebenenfalls im Gemisch mit äußeren Trennmitteln, verwenden, wie sie z. B. aus den DE-Offenlegungsschriften 2 121 670 und 2 307 589 bekanntgeworden sind.

Selbstverständlich können aber auch Schaumstoffe durch Blockverschäumung oder nach dem an sich bekannten Doppeltransportbandverfahren hergestellt werden.

Die nach der Erfindung erhältlichen Produkte finden z. B. als Dämmplatten in der Bauindustrie sowie zur Isolierung von Rohrleitungen (Rohrhalbschalen) und zur Bodenisolierung von Tiefkühlhäusern Anwendung.

### Beispiel 1
### Tris-natriumacetyl-hexyhydrotriazin

In 100 ml Wasser werden 40 g NaOH und 75 g Glycin gelöst, 30 g Paraformaldehyd zugesetzt, gelöst und das Wasser mit Toluol ausgekreist, abgesaugt und getrocknet. Die Aminalstruktur wurde über $^1$HNMR gesichert.

| | | | | |
|---|---|---|---|---|
| Analyse für $Na_3C_9H_{12}N_3O_6$ | ber. | C 33,4% | H 3,7% | N 13,0% |
| | gef. | C 32,0% | H 4,5% | N 12,5% |

### Beispiel 2
### Tris-kaliumacetyl-hexahydrotriazin

In 100 ml Wasser werden 56 g KOH und 75 g Glycin gelöst, 30 g Paraformaldehyd zugesetzt, gelöst und das Wasser mit Toluol ausgekreist, abgesaugt und getrocknet.
Die Aminalstruktur wurde über $^1$HNMR gesichert.

| | | | | |
|---|---|---|---|---|
| Analyse für $K_3C_9H_{12}N_3O_6$ | ber. | C 29,1% | H 3,2% | N 11,2% |
| | gef. | C 30,0% | H 4,2% | N 10,5% |

### Beispiel 3
### Bis-natriumacetyl-$\gamma$-dimethylaminopropyl-hexahydrotriazin

In 100 ml Wasser werden 40 g NaOH, 75 g Glycin, 51 g $\gamma$-Dimethylaminopropylamin und 45 g Paraformaldehyd gegeben; das Wasser wird anschließend mit Toluol ausgekreist. Man saugt ab und trocknet.

| | | | | |
|---|---|---|---|---|
| Analyse für $Na_2C_{12}H_{22}N_4O_4$ | ber. | C 39,6% | H 6,05% | N 15,3% |
| | gef. | C 38,0% | H 5,9% | N 14,7% |

## Beispiel 4
### Tris-natriumpropionyl-hexahydrotriazin

In 100 ml Wasser werden 40 g NaOH und 89 g β-Aminopropionsäure gelöst, mit 30 g Paraformaldehyd versetzt und anschließend das Wasser aceotrop ausgekreist. Man saugt ab und trocknet.

Analyse für $Na_3C_{12}H_{18}N_3O_6$ ber. C 39,4% H 4,9% N 11,0%
gef. C 39,8% H 5,7% N 10,5%

## Beispiel 5
### Tris-kaliumpropionyl-hexahydrotriazin

In 100 ml Wasser werden 56 g KOH und 89 g β-Aminopropionsäure gelöst, mit 30 g Paraformaldehyd versetzt und anschließend das Wasser aceotrop ausgekreist. Man saugt ab und trocknet.

Analyse für $K_3C_{12}H_{18}N_3O_6$ ber. C 34,6% H 4,3% N 10,1%
gef. C 35,8% H 5,5% N 9,3%

## Beispiel 6
### Tris-kaliumbutyryl-hexahydrotriazin

In 100 ml Wasser werden 56 g KOH, 103 g γ-Aminobuttersäure und 30 g Paraformaldehyd gelöst; anschließend wird das Wasser mit Toluol ausgekreist. Man saugt ab und trocknet.

Analyse für $K_3C_{15}H_{24}N_3O_6$ ber. C 39,2% H 5,2% N 9,2%
gef. C 42,8% H 6,4% N 10,0%

## Beispiel 7
### Tris-kaliumcapropyl-hexahydrotriazin

In 100 ml Wasser werden 56 g KOH, 131 g ω-Aminocapronsäure und 30 g Paraformaldehyd gelöst; anschließend wird das Reaktionswasser mit Toluol ausgekreist. Man saugt ab und trocknet.

Analyse für $K_3C_{11}H_{21}N_3O_6$ ber. C 48,0% H 4,0% N 8,0%
gef. C 47,1% H 5,5% N 7,2%

## Beispiel 8

Es wird eine Polyolmischung aus 17 Gew.-Tln. eines Zuckerpolyäthers (Hydroxylzahl 450, Startergemisch Zucker/Wasser [Wassergehalt von 15 bis 35%] Propylenoxid), 4 Gew.-Tln. eines Aminopolyäthers (Hydroxylzahl 650), hergestellt aus Äthylendiamin und Propylenoxid, 10 Gew.-Tln. eines Polyesters (Funktionalität 3, Hydroxylzahl 200) aus Adipinsäure, Phthalsäure, Glycerin und Diäthylenglycol), 1 Gew.-Tl. eines handelsüblichen Siliconstabilisators und 14 Gew.-Tln. Trichloräthylphosphat hergestellt.

Die folgende Tabelle faßt die Verschäumungsergebnisse zusammen, die mit verschiedenen Katalysatoren erzielt wurden.

Hierzu wurden jeweils 40 Gew.-Tl. der genannten Mischung mit 20 Gew.-Tln. Trichlorfluormethan, der in der Tabelle angegebenen Katalysatormenge und 100 Gew.-Tln. eines Präpolymeren aus 95 Gew.-Tln. rohem 4,4'-Diphenylmethandiisocyanat hergestellt durch Formaldehyd-Anilin-Kondensation und nachfolgende Phosgenierung, NCO Gehalt 31 Gew.-% und 5 Gew.-Tln. Tetrapropylenglycol 20 Sec. intensiv verrührt.

Tabelle 1

| | Katalysator gemäß Beispiel 1 | 2 | 4 | 3 | 25%ige Kaliumacetatlösung in Diäthylenglykol |
|---|---|---|---|---|---|
| Menge/gem. Beisp. | 3 | 1 | 1,7 | 1,8 | 0,7 |
| Startzeit | 155 | 200 | 110 | 120 | 130 |
| Abbindezeit | 300 | 600 | 300 | 300 | 300 |
| Schrumpf | nein | nein | nein | nein | ja |

Die Versuche zeigen, daß die erfindungsgemäßen Katalysatoren bei langen Reaktionszeiten vorteilhaft gegenüber dem üblicherweise verwendeten Kaliumacetat sind, da mit ihnen aktivierte Schaumstoffe nicht schrumpfen. Es werden vorzugsweise 20%ige Lösungen der erfindungsgemäßen Katalysatoren in Diäthylenglykol verwendet.

## Beispiel 9

Die Arbeitsweise von Beispiel 8 wurden beibehalten. 2 Gew.-Tl. der Katalysatorlösung gemäß Beispiel 2 (20%ige in Diäthylenglykol) werden mit den in Tabelle 2 genannten Polyurethan-Katalysatoren kombiniert.

Tabelle 2

| Katalysatoren | A | B | C | D | E |
|---|---|---|---|---|---|
| Menge | 0,2 | 0,1 | 1 | 0,2 | 0,2 |
| Startzeit | 40 | 60 | 35 | 60 | 23 |
| Abbindezeit | 120 | 160 | 140 | 160 | 90 |

A = Dimethylcyclohexylamin
B = Tris-(dimethylaminopropyl)-hexahydrotriazin
C = 2,4,6-Tris-(dimethylaminomethyl-)phenol
D = Triäthylamin
E = Dimethyläthanolamin/Dibutylzinndilaurat (Gew.-Verhältnis 3 : 1)

Keiner der Schaumstoffe zeigte Schrumpferscheinungen. Bei Verwendung einer 25%igen Kaliumacetatlösung in Verbindung mit den aufgeführten Co-Katalysatoren A bis E treten Schrumpferscheinungen auf.

**Patentansprüche**

1. Hexahydrotriazincarboxylate der allgemeinen Formel

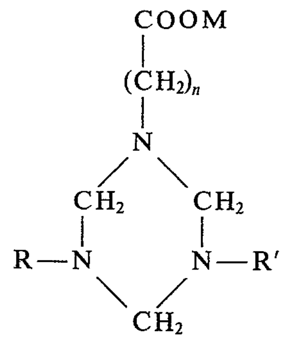

in der

R und R' gleich oder verschieden sein können und einen Rest

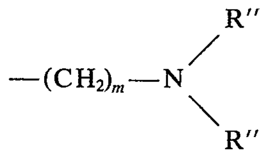

oder einen Rest

$$-(CH_2)_n-COOM$$

bedeuten,

R'' einen $C_1-C_3$-Alkylrest, vorzugsweise einen Methylrest,
m eine ganze Zahl von 2 bis 6, vorzugsweise 2—4
n eine ganze Zahl von 1 bis 10, vorzugsweise 1—4, und
M ein Alkalimetall, vorzugsweise Na oder K, oder $NR_4'''$ (R''' H oder $C_1-C_4$-Alkyl) darstellt.

2. Verfahren zur Herstellung von Hexahydrotriazincarboxylaten gemäß Anspruch 1, dadurch gekennzeichnet, daß man äquimolare Mengen an Formaldehyd und den Verbindungen

$$H_2N-(CH_2)_n-COOM \qquad H_2N-R$$

und

$$H_2N-R'$$

in denen R, R' und M die im Anspruch 1 genannten Bedeutungen haben, miteinander umsetzt.

3. Verwendung der Hexahydrotriazincarboxylate gemäß Anspruch 1 als Katalysatoren zur Herstellung von Polyisocyanurat-Kunststoffen, gegebenenfalls unter Mitverwendung von bekannten Polyurethan-Katalysatoren.

**Claims**

1. Hexahydrotriazine carboxylates of the general formula:

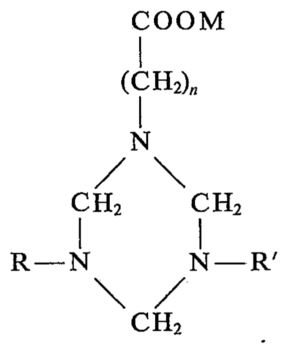

in which

R and R′ may be the same or different and denote a radical

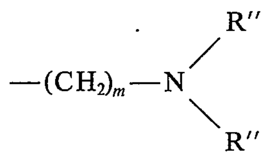

or a radical

$$-(CH_2)_n-COOM$$

R″ represents a $C_1-C_3$ alkyl radical, preferably a methyl radical,
m represents an integer of from 2 to 6, preferably 2 to 4
n represents an integer of from 1 to 10, preferably 1 to 4, and
M represents an alkali metal, preferably Na or K or $NR_4'''$ (R‴ H or $C_1-C_4$ alkyl).

2. Process for the production of hexahydrotriazine carboxylates according to claim 1, characterised in that equimolar quantities of formaldehyde and the compounds

$$H_2N-(CH_2)_n-COOM \qquad H_2N-R$$

and

$$H_2N-R'$$

in which R, R′ and M have the meanings mentioned in claim 1, are reacted with each other.

3. Use of the hexahydrotriazine carboxylates according to claim 1 as catalysts for the production of polyisocyanurate plastics, optionally also using known polyurethane catalysts.

**Revendications**

1. Hexahydrotriazine-carboxylates de formule générale

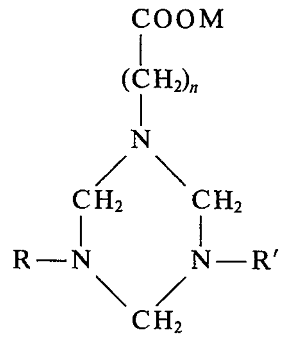

dans laquelle R et R′ peuvent être identiques ou différents et représentent un reste

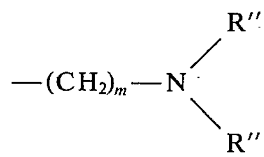

ou un reste

$$-(CH_2)_n-COOM$$

R″ représente un reste alkyle en $C_1-C_3$, de préférence méthyle, m est un entier de 2 à 6, de préférence 2—4, n est un entier de 1 à 10, de préférence 1—4, et M est un métal alcalin, de préférence Na ou K, ou bien $NR_4'''$ (R‴ = H ou alkyle en $C_1-C_4$).

2. Procédé pour la préparation d'hexahydrotriazine-carboxylates selon la revendication 1, caractérisé en ce que l'on fait réagir ensemble des quantités équimolaires de formaldéhyde et des composés

$$H_2N-(CH_2)_n-COOM \qquad H_2N-R$$

et

$$H_2N-R'$$

dans lesquels R, R′ et M ont les significations indiquées à la revendication 1.

3. Utilisation des hexahydrotriazine-carboxylates selon la revendication 1 comme catalyseurs pour la préparation de matières plastiques de polyisocyanurates, éventuellement avec utilisation combinée de catalyseurs pour polyuréthannes connus.